# DEMANDE DE BREVET EUROPEEN

(11) **EP 4 799 680 A1**
(43) Date de publication de la demande: **02.09.2026**
(21) Numéro de dépôt: 26156474.4
(22) Date de dépôt: 04.02.2026
(51) Int. Cl.: A61N 5/06

(54) **DISPOSITIF DE PHOTOTHÉRAPIE**

(30) Priorité: 06.02.2025 FR 2501239
(71) Demandeur: Lucibel SA, 76770 Le Houlme (FR)
(72) Inventeur: GROS-DAILLON, Thibault, 76590 Criquetot-sur-Longueville (FR); MOA, Yacine, 76510 Saint Nicolas d'Aliermont (FR)
(74) Mandataire: Martin, Marie-Aude

(57) **Abrégé**

Le dispositif (10) de traitement par photothérapie comprenant une armature (12) de support de configuration globalement plane et comprenant une source de lumière (18) comprenant une pluralité d'éléments lumineux (20) portés par l'armature (12). Selon l'invention, l'armature (12) étant formée par une pluralité de branches longitudinales (22) et de branches transversales (24) disposées de manière à former la configuration plane, les branches longitudinales (22) étant reliées entre elles par les branches transversales (24), ladite armature (12) comprenant une face frontale d'émission (14) de la lumière et une face dorsale (16) située du côté opposé.

## Description

L'invention concerne un dispositif de photothérapie destiné à traiter le corps humain en émettant de la lumière thérapeutique afin de stimuler des processus biologiques et d'améliorer le bien-être général des individus. Plus précisément, ce dispositif est conçu pour traiter une large portion du corps, voire sa totalité, en émettant des lumières de différentes longueurs d'onde, adaptées à des fins thérapeutiques telles que la régénération cellulaire, la réduction de l'inflammation, le soulagement de la douleur ou le traitement de diverses affections dermatologiques.

Ce dispositif trouve son application dans le domaine des soins esthétiques, notamment pour le traitement de la peau du visage ou du corps par photothérapie, par exemple à l'aide de diodes électroluminescentes. Il permet ainsi d'améliorer l'apparence de la peau en agissant sur des problèmes comme les rides, les cicatrices, l'acné ou les vergetures, sans recourir à des méthodes invasives ou chimiques. La lumière émise, qu'elle soit rouge, bleue ou jaune, offre des bienfaits reconnus en matière de soins de la peau et de bien-être, en stimulant des récepteurs cellulaires spécifiques et en favorisant la récupération du corps.

Pour ce faire, des dispositifs utilisant des sources lumineuses, notamment des diodes électroluminescentes, sont utilisés pour émettre de la lumière sur la surface de la peau. Le dispositif de photothérapie de l'invention est conçu pour offrir une exposition uniforme à la lumière, couvrant une large surface corporelle et offrant ainsi un confort optimal tout en garantissant une efficacité thérapeutique élevée.

L'objectif de l'invention est de fournir une solution permettant une exposition à la lumière spécifique de manière rapide et efficace, sans perturber les activités quotidiennes des utilisateurs. Ainsi, cette approche se veut pratique et accessible, pouvant facilement s'intégrer dans la routine des utilisateurs et maximisant les effets bénéfiques de la photo-biomodulation et de la photothérapie.

### Description de l'invention

A cet effet l'invention a pour objet un dispositif de traitement par photothérapie comprenant une armature de support de configuration globalement plane et comprenant une source de lumière comprenant une pluralité d'éléments lumineux portés par l'armature, **caractérisé en ce que** l'armature étant formée par une pluralité de branches longitudinales et de branches transversales disposées de manière à former la configuration plane, les branches longitudinales étant reliées entre elles par les branches transversales, ladite armature comprenant une face frontale d'émission de la lumière et une face dorsale située du côté opposé et en ce que le dispositif comprend au moins un moyen de fixation libérable sur une surface sensiblement plane, conçu pour pivoter autour d'un axe horizontal ou vertical de l'armature, de manière à orienter indifféremment la face d'émission ou la face dorsale contre ladite surface.

Le dispositif selon l'invention peut être monté sur n'importe quelle paroi, qu'elle soit transparente ou non grâce à la fixation réversible de l'armature. Ce dispositif de photothérapie permet en outre à l'utilisateur de mettre à profit son temps d'hygiène pour effectuer une séance de traitement de photothérapie.

Le dispositif de l'invention peut comprendre l'une ou l'autre des caractéristiques suivantes.

Selon un mode de réalisation préféré de l'invention, le moyen de fixation est monté dans une partie médiane d'au moins une des branches transversales.

Selon un mode de réalisation préféré de l'invention, le module d'éclairage a une fonction de rigidification de l'armature dans une direction longitudinale.

Selon un mode de réalisation préféré de l'invention, le moyen de fixation comprend une ventouse montée pivotante autour d'une branche transversale.

Selon un mode de réalisation préféré de l'invention, les branches transversales sont fabriquées dans un matériau déformable autorisant l'enroulement de l'armature selon la direction transversale.

Selon un mode de réalisation préféré de l'invention, des branches longitudinales sont conçues pour être plus rigides que les branches transversales.

Selon un mode de réalisation préféré de l'invention, les branches transversales sont réalisées dans un matériau à base d'un élastomère, par exemple un thermoplastique élastomère à base d'uréthane.

Selon un mode de réalisation préféré de l'invention, les branches longitudinales portent les éléments lumineux qui sont disposés dans le sens de la longueur dans un module d'éclairage.

Selon un mode de réalisation préféré de l'invention, les éléments lumineux comprennent des diodes électroluminescentes équipées de système optiques permettant de diriger et focaliser leur faisceau lumineux selon un angle d'émission prédéfini, par exemple compris entre 20° et 40°, de préférence entre 25° à 35°.

Selon un mode de réalisation préféré de l'invention, le système optique est unitaire associé à chaque diode électroluminescente.

L'invention sera bien comprise et ses avantages apparaîtront mieux, à la lecture de la description détaillée qui suit, de modes de réalisation représentés à titre d'exemples non limitatifs. La description se réfère aux dessins annexés sur lesquels :
[Fig. 1] La figure 1 est une vue en perspective d'un dispositif de traitement par photothérapie montée sur une paroi d'une douche dans une salle de bains selon un mode de réalisation préféré de l'invention.
[Fig. 2] La figure 2 est une vue en perspective du dispositif de la figure 1.
[Fig. 3] La figure 3 est une vue de face du dispositif de la figure 2.
[Fig. 4] La figure 4 est une vue partielle et en perspective à échelle agrandie du dispositif de la figure 2.
[Fig. 5] La figure 5 est une vue en coupe longitudinale du dispositif de la figure 4.

### Description détaillée de l'invention

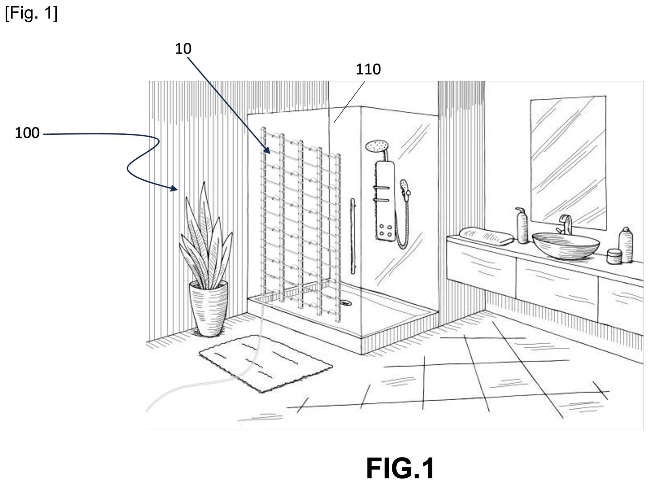

On a représenté de façon schématique sur la figure 1 un dispositif de traitement par photothérapie selon l'invention qui est installé dans une salle de bain telle qu'illustrée sur cette figure. Dans la suite de la description, le dispositif est désigné par la référence générale 10.

Comme illustré sur la figure 1, le dispositif 10 est par exemple conçu pour être monté sur une paroi 112 d'une douche transparente 110 d'une salle de bain 100.

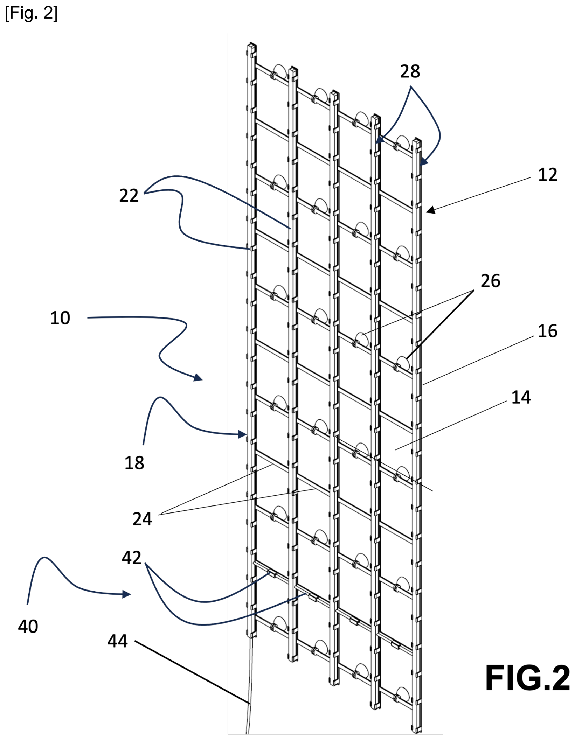

Sur la figure 2, le dispositif 10 comprend une armature 12 de support de configuration globalement plane. Comme cela est illustré, le dispositif 10 comprend également une source de lumière 18. La source de lumière 18 comprend une pluralité d'éléments lumineux 20 qui sont portés par l'armature 12. La source 18 émet par exemple une lumière comprise entre 630 et 640 nanomètres qui est connue pour présenter une efficacité thérapeutique. Bien entendu, l'invention ne se limite pas à cette plage de longueurs d'onde. La source de lumière 18 peut être configurée pour émettre dans différentes bandes de longueurs d'onde du spectre visible mais également de l'infrarouge.

L'armature 12 comprend de préférence une face frontale d'émission 14 de la lumière et une face dorsale 16 située du côté opposé.

L'armature 12 est formée par une pluralité de branches longitudinales 22 et de branches transversales 24 disposées de manière à former la configuration plane, les branches longitudinales 22 étant reliées entre elles par les branches transversales 24. Les branches longitudinales 22 et les branches transversales 24 forment ensemble de préférence un quadrillage ou une grille 12.

De préférence, le dispositif 10 comprend au moins un moyen 26 de fixation libérable à une surface sensiblement plane 112, conçu pour pivoter autour d'un axe horizontal ou vertical de l'armature 12, de manière à orienter indifféremment la face d'émission 14 ou la face dorsale 16 contre ladite surface 112.

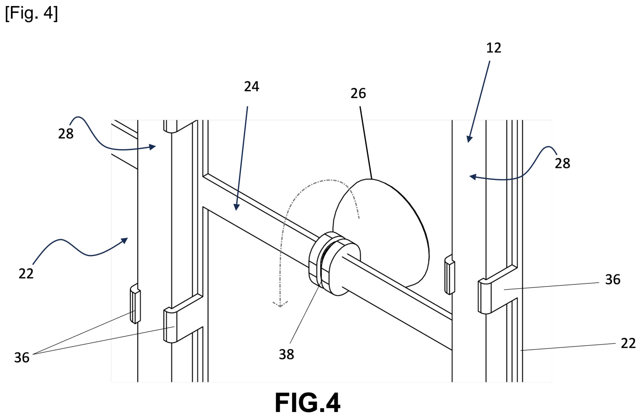

Comme illustré sur la figure 4, le moyen de fixation 26 est monté par exemple dans une partie médiane d'au moins une des branches transversales 24. A cet effet, la branche transversale 24 comprend dans sa partie médiane un moyen de réception 38 en pivotement du moyen de fixation 26, par exemple en forme de paliers de rotation. Le moyen de fixation 26 comprend par exemple une ventouse montée pivotante autour des branches transversales 24. Le moyen de fixation 26 est situé de préférence de manière centrale dans la partie médiane de l'une des branches transversales 24, assurant ainsi une répartition équilibrée des forces exercées lors de l'utilisation.

De préférence, la ventouse 26 est montée de manière pivotante autour de l'axe des branches transversales 24. Cette configuration permet à la ventouse 26 de se positionner de façon réversible autour de l'axe de rotation, en basculant d'une face à l'autre, par exemple à 180°, offrant ainsi une flexibilité et une adaptabilité accrues lors de l'application du dispositif 10 sur une surface plane d'une paroi qu'elle soit transparente ou non.

Les ventouses 26 sont montées sur la branche transversale 24 par l'intermédiaire d'un pivot 27, ce qui permet de les orienter vers la paroi 110 de la douche, que le dispositif 10 soit installé à l'intérieur ou à l'extérieur de celle-ci tout en permettant une orientation de la face lumineuse 14 vers l'intérieur de la douche.

De façon connue en soi, une ventouse est un dispositif de fixation temporaire utilisant le principe de la succion pour créer un vide entre la ventouse et la surface, assurant ainsi une adhérence sans adhésifs. Elle est généralement composée d'un matériau flexible et élastique, permettant une utilisation sur des surfaces lisses et non poreuses. Elle est généralement fabriquée à partir de matériaux flexibles, comme le silicone, pour assurer une bonne étanchéité et une fixation stable.

De préférence, les branches transversales 24 sont fabriquées dans un matériau déformable, permettant à l'armature 12 de s'enrouler librement selon la direction transversale. Cette déformabilité offre à l'armature 12 une certaine flexibilité, facilitant ainsi l'adaptation aux contraintes et aux formes spécifiques lors de son utilisation.

En revanche, les branches longitudinales 22 sont conçues de préférence pour être plus rigides que les branches transversales 24. Cette rigidité des branches longitudinales 22 joue un rôle essentiel en stabilisant le processus d'enroulement de l'armature 12, en limitant les déformations excessives et en assurant une structure plus stable pendant l'utilisation dans la configuration plane.

Grâce à cette configuration, les branches longitudinales rigides 22 contribuent à maintenir l'intégrité de l'ensemble du dispositif 10, en empêchant les branches transversales 24 de se déformer de manière incontrôlée, tout en permettant à l'armature 12 de s'adapter aux différentes formes sans compromettre sa stabilité générale. Ce mécanisme optimise à la fois la flexibilité et la solidité, en garantissant un enroulement contrôlé et une performance stable du dispositif 10 dans son ensemble.

De préférence, les branches longitudinales 22 supportent les éléments lumineux 20 qui sont disposés dans le sens de la longueur, orientés selon une des faces de l'armature 12, ici la face frontale 14.

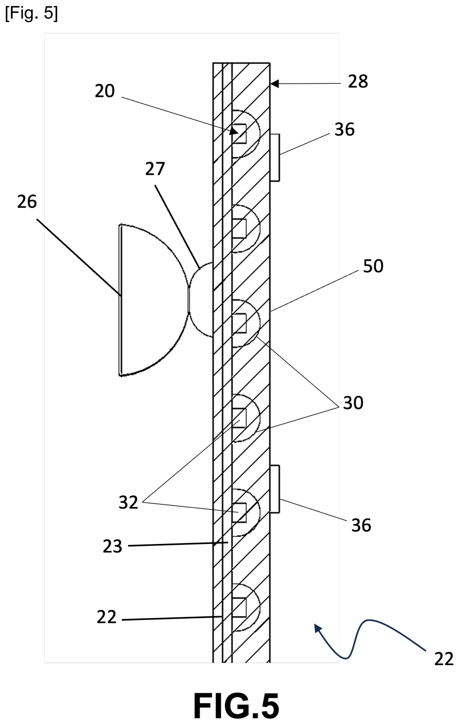

Les éléments lumineux 20 sont rapportés de préférence sous forme d'un module d'éclairage longitudinal 28, illustré en détail sur les figures 4 et 5. Le module d'éclairage 28 comprend de préférence une barrette 23 d'éclairage longitudinale supportant les éléments lumineux 20. Les modules d'éclairage 28 comprennent encore de préférence un capot de diffusion de la lumière 50 monté au-dessus de la barrette 23.

Les éléments lumineux 20 comprennent par exemple des diodes électroluminescentes 32 équipées de système optiques 34 permettant de diriger et focaliser leur faisceau lumineux selon une angle d'émission prédéfini, par exemple compris entre 20° et 40°, de préférence entre 25° à 35°. Dans l'exemple illustré sur la figure 5, le système optique 34 est unitaire et est associé à chaque diode électroluminescente 32.

Par exemple, l'armature 12 comprend une grille ou un quadrillage réalisé au moins partiellement en matériau souple, offrant ainsi une flexibilité et une légèreté qui permettent à l'ensemble de s'adapter facilement à des configurations variées. Cette structure souple permet à l'armature 12 de se déformer et de se modeler selon les besoins spécifiques de l'application. Cette grille peut être réalisée par exemple de façon hybride à la fois dans un matériau rigide pour les branches longitudinales 22 et dans un matériau plus flexible pour les branches transversales 24.

En outre, l'armature 12 peut par exemple être réalisée dans un matériau souple non seulement pour les branches longitudinales 22 mais également pour les branches transversales 24. Dans ce cas, pour garantir la stabilité et la solidité de l'armature 12 dans ses directions longitudinales, les modules d'éclairage 28 peuvent être utilisés comme rigidificateurs de l'armature 12. Dans ce cas, l'armature 12 peut comprendre une grille réalisée dans un même matériau souple qui est rigidifiée par les modules d'éclairage 28 dans la direction longitudinale.

Les modules d'éclairage 28 ont de préférence une fonction de rigidification des branches longitudinales 22. Ainsi, ces modules d'éclairage 28, tout en assurant leur fonction primaire d'éclairage, jouent également un rôle crucial dans le renforcement de l'armature 12. En étant intégrés à l'armature 12, ils apportent une rigidité nécessaire qui limite les déformations dans la direction longitudinale, tout en maintenant une flexibilité optimale dans les directions transversales. Ce système hybride entre souplesse et rigidité permet de combiner la légèreté et l'adaptabilité du matériau souple avec la stabilité et la résistance accrues offertes par les modules d'éclairage 28, garantissant ainsi la durabilité et la performance du dispositif.

Comme cela est illustré sur la figure 4, les modules 28 sont maintenus en place par clipsage à l'armature 12, grâce à des griffes de fixation 36.

Ces griffes 36 sont réalisées par exemple dans un matériau rigide par moulage par injection et la grille 12 est réalisée dans un matériau plus souple par surmoulage sur les griffes 36 rigides. La ventouse 26 est montée par exemple sur un pivot réalisé également dans un matériau rigide. Le matériau rigide pour les griffes 36 et les pivots 27 comprend essentiellement par exemple de l'ABS (Acrylonitrile butadiène Styrène). Mais d'autres matériaux rigides peuvent convenir tels que le polycarbonate, le polypropylène, le polyéthylène haute densité, le polyamide, le polyoxyméthylène (POM). Ces matériaux peuvent avantageusement être combinés avec d'autres plastiques souples lors du processus de surmoulage, afin d'obtenir des pièces présentant des propriétés particulières comme la flexibilité sur certaines zones et la rigidité sur d'autres.

Par exemple, les branches transversales 24 sont réalisées dans un matériau à base d'un élastomère, par exemple un thermoplastique élastomère à base d'uréthane. Mais d'autres matériaux pourraient convenir tels que par exemple le silicone.

Dans l'exemple illustré sur la figure 5, les différents modules d'éclairage 28 sont reliés électriquement entre eux par un réseau électrique 40 comprenant des connecteurs 42 étanches permettant de garantir la sécurité de l'utilisateur lorsque le dispositif 10 est utilisé près d'une source d'eau. Le réseau électrique 40 comprend encore un câble électrique 44 de raccordement des modules d'éclairage 28 à une source d'alimentation électrique (non représentée).

Par ailleurs, par exemple, les dimensions de l'armature 12 dans une configuration déroulée sont comprises entre 50 à 150 centimètres selon la direction longitudinale et entre 25 et 75 centimètres selon la direction transversale.

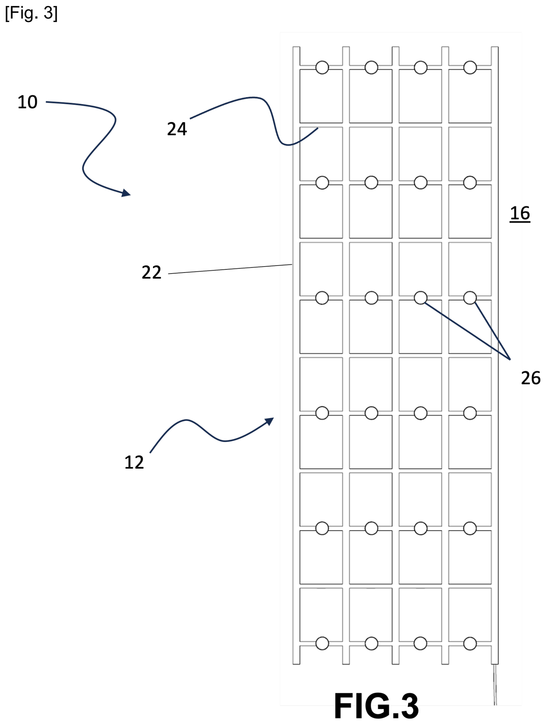

On va maintenant décrire les principaux aspects d'un dispositif de photothérapie selon l'invention en référence aux figures 1 à 5.

Initialement, l'armature 12 est dans une configuration enroulée dans le sens de la largeur ce qui permet son rangement pratique dans un volume réduit. Puis, l'armature 12 est dépliée dans un sens inverse au sens d'enroulement.

L'utilisateur positionne le dispositif 10 à l'extérieur de la douche et le fixe grâce aux moyens de fixation 26 libérales sur la surface plane de la paroi de douche 112 par la face frontale 14 de l'armature 12 en faisant pivoter les moyens de fixation 26 du même côté que le côté d'émission de la source lumineuse 18.

Si l'utilisateur décide de la positionner sur un miroir ou l'intérieur d'une douche, il peut alors faire pivoter les moyens de fixation 26 pour permettre une fixation par la face dorsale 16 et non plus par la face frontale 14 de l'armature 12.

L'utilisateur active alors le dispositif 10 de photothérapie pour que ce dernier émette de la lumière en direction de son corps pendant toute la durée de la douche. Ceci lui permet de mettre à profit son temps d'hygiène corporelle pour une séance de traitement de photothérapie.

Bien entendu, d'autres modes de réalisation sont envisageables sans sortir du cadre de l'invention. Ainsi, diverses modifications peuvent être apportées par l'homme du métier à l'invention qui vient d'être décrite à titre d'exemple.

## Revendications

1. Dispositif (10) de traitement par photothérapie comprenant une armature (12) de support de configuration globalement plane et comprenant une source de lumière (18) comprenant une pluralité d'éléments lumineux (20) portés par l'armature (12), **caractérisé en ce que** l'armature (12) étant formée par une pluralité de branches longitudinales (22) et de branches transversales (24) disposées de manière à former la configuration plane, les branches longitudinales (22) étant reliées entre elles par les branches transversales (24), ladite armature (12) comprenant une face frontale d'émission (14) de la lumière et une face dorsale (16) située du côté opposé et **en ce qu'**il comprend au moins un moyen (26) de fixation libérable sur une surface sensiblement plane, conçu pour pivoter autour d'un axe horizontal ou vertical de l'armature (12), de manière à orienter indifféremment la face d'émission (14) ou la face dorsale (16) contre ladite surface.

2. Dispositif (10) selon la revendication précédente, dans lequel le moyen de fixation (26) est monté dans une partie médiane d'au moins une des branches transversales (24).

3. Dispositif (10) selon l'une quelconque des revendications précédentes, dans lequel le moyen de fixation (26) comprend une ventouse montée pivotante autour d'une branche transversale (24).

4. Dispositif (10) selon l'une quelconque des caractéristiques précédentes, dans lequel les branches transversales (24) sont fabriquées dans un matériau déformable autorisant l'enroulement de l'armature (12) selon la direction transversale.

5. Dispositif (10) selon l'une quelconque des revendications précédentes, dans lequel des branches longitudinales (22) sont conçues pour être plus rigides que les branches transversales (24).

6. Dispositif (10) selon l'une quelconque des revendications précédentes, dans lequel les branches transversales (24) sont réalisées dans un matériau à base d'un élastomère, par exemple un thermoplastique élastomère à base d'uréthane.

7. Dispositif (10) selon l'une quelconque des revendications précédentes, dans lequel les branches longitudinales (22) portent les éléments lumineux (20) qui sont disposés dans le sens de la longueur dans un module d'éclairage (28).

8. Dispositif (10) selon la revendication précédente, dans lequel le module d'éclairage (28) a une fonction de rigidification de l'armature (12) dans une direction longitudinale.

9. Dispositif (10) selon l'une quelconque des revendications précédentes, dans lequel les éléments lumineux (20) comprennent des diodes électroluminescentes (32) équipées de système optiques (34) permettant de diriger et focaliser leur faisceau lumineux selon un angle d'émission prédéfini, par exemple compris entre 20° et 40°, de préférence entre 25° à 35°.

10. Dispositif (10) selon la revendication précédente, dans lequel le système optique (34) est unitaire associé à chaque diode électroluminescente (32).
